# EUROPÄISCHE PATENTSCHRIFT

(11) **EP 1 639 957 B1**
(45) Veröffentlichungstag und Bekanntmachung des Hinweises auf die Patenterteilung: **27.01.2010**
(21) Anmeldenummer: 04028158.6
(22) Anmeldetag: 26.11.2004
(51) Int. Cl.: A61B 18/14

(54) **Frequenz-Ablationsgerät**
Frequency ablation apparatus
Appareil d'ablation à fréquence

(30) Priorität: 24.09.2004 DE 202004014924 U
(43) Veröffentlichungstag der Anmeldung: 29.03.2006
(73) Patentinhaber: Möller Medical GmbH, 36043 Fulda (DE)
(72) Erfinder: Traxler, Christoph, 36100 Petersberg (DE); Schröter, Werner, 36137 Grossenlüder (DE)
(74) Vertreter: Grünecker, Kinkeldey, Stockmair & Schwanhäusser Anwaltssozietät

(56) Entgegenhaltungen:
- WO-A1-03/020144
- US-A1- 2004 158 239
- US-B1- 6 575 967

## Beschreibung

Die vorliegende Erfindung betrifft ein Frequenz-Ablationsgerät mit welchem krankes Gewebe koagulierbar ist.

Aus der US 6,648,882 B1 ist ein Ablationsgerät mit zwei schirmartigen Elektrodenanordnungen bekannt. Die Elektrodenanordnungen werden durch eine Mehrzahl federelastisch verformbarer Arme ausgebildet Die Arme treten jeweils aus einem Führungsrohr aus, in welches sie vollständig zurückziehbar sind. Die Führungsrohre sind koaxial zueinander angeordnet

Das Ablationsgerät ist so gewählt, dass sich die konkaven Seiten der beiden Elektrodenanordnungen einander gegenüberliegen, um ein für die Behandlung am besten geeignetes, gleichmäßiges homogenes elektrisches Feld zu erhalten.

Das Bewegen der Arme relativ zu den Führungsrohren, also das Ausbilden und Einziehen der Elektrodenanordnungen, erfolgt bowdenzugartig mit einem elektrischen Leiter. Mit einem Gewindemechanismus in einem Griff des Ablationsgerätes können die Leiter in zueinander entgegengesetzten Richtungen bewegt werden. Dies ist erforderlich, da die konkaven Seiten der Elektrodenanordnungen einander gegenüberliegend vorgesehen sind. Entsprechend aufwendig und voluminös ist das Ablationsgerät ausgebildet Je größer jedoch der Bereich des Ablationsgerätes ist, welcher in den zu behandelnden Körper einzuführen ist, desto größer sind die durch das Einführen des Ablationsgerätes hervorgerufenen Verletzungen und je größer ist das Risiko, Gefäße und Nervenbahnen zu beschädigen. Außerdem ist ein derartiges Ablationsgerät in seinen Bewegungsmöglichkeiten in dem Körper eingeschränkt.

Aus der WO03/020144 ist ein Ablationsgerät mit zwei aus Drahten gebildeten Elektrodenanordnungen bekannt. Der Abstand der Elektrodenanordnungen ist fix. Für verschiedene Tumorgrößen müssen verschiedene Ablationsgeräte mit verschiedenen Abständen zwischen den Elektrodenanordnungen verwendet werden.

Der vorliegenden Erfindung liegt die Aufgabe zugrunde, ein Frequenz-Ablationsgerät zu schaffen, welches auf möglichst einfache Weise bei kompakten Abmessungen einen einfachen Aufbau hat und trotzdem leicht zu handhaben ist.

Die Aufgabe wird erfindungsgemäß gelöst mit einem Frequenz-Ablationsgerät mit den Merkmalen des Anspruches 1.

Durch den rohrartigen Aufbau und das Vorsehen der federelastisch verformbaren Arme an den Enden des ersten und dritten Rohres wird ein einfacher und kompakter Aufbau erreicht. Dies kommt der Flexibilität des erfindungsgemäßen Ablationsgerätes in dem zu behandelnden Körper zugute, wobei der Körper beim Zuführen und Anwenden des Ablationsgerätes weniger stark verletzt wird und auch das Risiko, Gefäße oder Nervenbahnen zu verletzten, verringert ist.

Durch das einstückige Ausbilden der Arme des ersten und/oder dritten Rohres mit ihrem jeweiligen Rohr sind die Arme mit ihrem jeweiligen Rohr integriert verbunden, wodurch das jeweilige Rohr noch kompakter ausgebildet werden kann. Dabei ist gleichzeitig die Herstellung des Rohres vereinfacht ist und der Kraftfluss zwischen den Armen und dem übrigen Teil des Rohres ist verbessert. Das Laserschneiden ermöglicht ein schnelles und präzises Ausbilden der Arme bei hoher Gestaltungsfreiheit. Wegen ihrer gespleißten Ausführung, also ihrem unmittelbaren Fortsetzen des übrigen Teiles ihres Rohres, ist der Kraftfluss zwischen den Armen und dem übrigen Teil ihres Rohres besonders gut bei guter Kompaktheit.

Durch die einfache Anordnung der koaxial ineinandergeführten Rohre sind diese in axialer Richtung einfach relativ zueinander bewegbar. Die Elektrodenanordnungen sind jeweils durch axiales Verschieben ihres Rohres in dem jeweiligen führenden Rohr unabhängig voneinander ausbildbar und wieder rückbildbar. Ferner lässt sich durch axiales Verschieben des zweiten Rohres relativ zu dem vierten Rohr der Abstand der Elektrodenanordnungen zueinander stufenlos und trotzdem leicht variieren. Hierdurch ist das Ablationsgerät schnell und auch unmittelbar vor Ort leicht an die Größe des zu behandelnden Gewebes anpassbar.

In einer besonders günstigen Ausführungsform der Erfindung können die Elektrodenanordnungen unterschiedlich bogenförmig gekrümmte Arme aufweisen und/oder wenigstens eine der Elektrodenanordnungen kann unterschiedlich bogenförmig gekrümmte Arme aufweisen. Durch das Vorsehen unterschiedlich bogenförmig gekrümmter Arme kann ein gut auf das Gewebe ausgerichtetes und verteiltes Feld zwischen den Elektrodenanordnungen erzeugt werden.

In einer besonders bevorzugten Ausführungsform der vorliegenden Erfindung kann eine Elektrodenanordnung wenigstens zwei schirmartige Teilelektrodenanordnungen aufweisen, wobei jede Teilelektrodenanordnung Arme eigener Ausgestaltung aufweist. Dies ermöglicht das Ausbilden eines homogeneren elektrischen und somit auch thermischen Feldes zwischen den Elektrodenanordnungen, da die die Struktur des elektrischen und somit thermischen Feldes deutlich beeinflussenden Endabschnitte der Arme gleichmäßiger verteilt sind. Durch ein homogeneres thermisches Feld kann das Gewebe gleichmäßiger koaguliert werden, wobei die Überlebenswahrscheinlichkeit von kranken Zellen in dem behandelten Gewebe verringert ist.

Besonders vorteilhaft können die Abschnitte der Arme der ersten Teilelektrodenanordnung einen anderen radialen Abstand von einer Längsachse ihres Rohres haben als die Arme der zweiten Teilelektrodenanordnung. Mit dieser Anordnung sind auf einfache Weise besonders gut homogenere Felder ausbildbar, wobei die Homogenität gut durch die Wahl der radialen Abstände der jeweiligen Endabschnitte der Arme von der Längsachse einstellbar ist.

In einer vorteilhaften Weiterbildung der Erfindung kann das Ablationsgerät eine Bedieneinheit aufweisen, mit welcher es von einer Ausgangsstellung, in welcher das erste und das dritte Rohr in ihr jeweiliges führendes Rohr zurückgezogen sind, über eine Zwischenstellung, in der die axiale Lage des zweiten Rohres relativ zum vierten Rohr eingestellt ist und sich das erste und dritte Rohr noch in den genannten zurückgezogenen Positionen befinden, in eine Anwendungsstellung bringbar ist, in welcher die Elektrodenanordnungen ausgebildet sind. Auf diese Weise unterstützt die Bedieneinheit ein einfaches und sicheres Einstellen der Rohre relativ zueinander, wodurch ein korrektes und damit sicheres Ausführen der Behandlung mit dem Ablationsgerät unterstützt wird.

Günstigerweise können die Elektrodenanordnungen erst dann schirmartig ausbildbar sein, wenn die Zwischenstellung erreicht worden ist. Hierdurch wird das Bedienen des Ablationsgerätes in der richtigen Reihenfolge sichergestellt.

In einer besonders bevorzugten Ausführungsform der vorliegenden Erfindung kann durch den Innenraum des ersten Rohres ein fließfähiges Medium leitbar und/oder eine Apparatur führbar sein. Dadurch können fließfähige Medien und/oder Apparaturen mit guter Präzision an die Stelle gebracht werden, an die das Ablationsgerät in dem zu behandelnden Körper gebracht ist, insbesondere zum distalen Öffnungsbereich des ersten Rohres. Dies erspart zudem zusätzliche Verletzungen des zu behandelnden Körpers und die damit verbundenen Risiken. Außerdem ist das Zuführen eines fließfähigen Mediums und/oder einer Apparatur schnell möglich, was die Möglichkeiten, an der zu behandelnden Stelle schnell einzugreifen, deutlich erhöht und darüber hinaus die Dauer der Behandlung insgesamt verkürzt.

Diese Ausführungsform der Erfindung ermöglicht auch das Führen des Ablationsgerätes entlang einer durch den Innenraum des ersten Rohres verlaufenden Apparatur. Auf diese Weise kann das Ablationsgerät präzise entlang eines durch die Apparatur definierten Pfades geführt werden.

Vorteilhafterweise kann die Apparatur ein Führungsdraht sein, an dem entlang das Ablationsgerät geführt in seine Verwendungsposition bringbar ist. Wegen seiner guten Flexibilität und Handhabbarkeit kann mit dem Führungsdraht leicht ein geeigneter Pfad zum Zuleiten des Ablationsgerätes an seine Verwendungsposition definiert werden. Das Zuführen des Ablationsgerätes auf diesem Pfad zu seiner Verwendungsposition erfolgt dann präzise mit guter Geschwindigkeit und verringertem Risiko für den zu behandelnden Körper.

In einer Weiterbildung der vorliegenden Erfindung kann die Apparatur eine Leitungsanordnung sein, durch welche ein fließfähiges Medium leitbar ist. Hiermit kann das Innere des ersten Rohres im Wesentlichen von einem Kontakt mit dem fließfähigen Medium freigehalten werden. Wird die Leitungsanordnung über das Ende des ersten Rohres hinausgeschoben, kann das fließfähige Medium auch in einen von der Position des Ablationsgerätes beabstandeten Bereich eingebracht werden.

Besonders günstig kann das fließfähige Medium ein Betäubungsmittel, ein Gewebeklebstoff oder ein Medikament sein. Wird ein Medikament oder Betäubungsmittel zugeführt, kann es seine Wirkung genau dort entfalten, wo es gebraucht wird, nämlich im Anwendungsbereich des Ablationsgerätes oder, bei Verwendung der Leitungsanordnung, in einem dazu beabstandeten Bereich. Beim Verwenden eines Gewebeklebstoffes kann dieser beispielsweise während des Rückzugs des Ablationsgerätes in das freiwerdende, verletzte Gewebe abgegeben werden, um dieses zu kleben und somit unmittelbar zu verschließen.

Eine Ausführungsform der vorliegenden Erfindung ist in der Zeichnung dargestellt und wird nachfolgend erläutert. Es zeigen:
- Figur 1: eine schematische Ansicht eines erfindungsgemäßen Frequenz-Ablationsgerätes in einer Ausgangsstellung,
- Figur 2: eine abschnittsweise, vergrößerte perspektivische Ansicht des Ablationsgerätes aus Figur 1,
- Figur 3: eine vergrößerte und in axialer Richtung gestauchte Schnittansicht des in Figur 2 gezeigten Abschnittes des Ablationsgerätes,
- Figur 4: eine abschnittsweise, vergrößerte perspektivische Ansicht des Ablationsgerätes in einer Zwischenstellung und
- Figur 5: eine abschnittsweise, vergrößerte, perspektivische Ansicht des Ablationsgerätes in einer Anwendungsstellung.

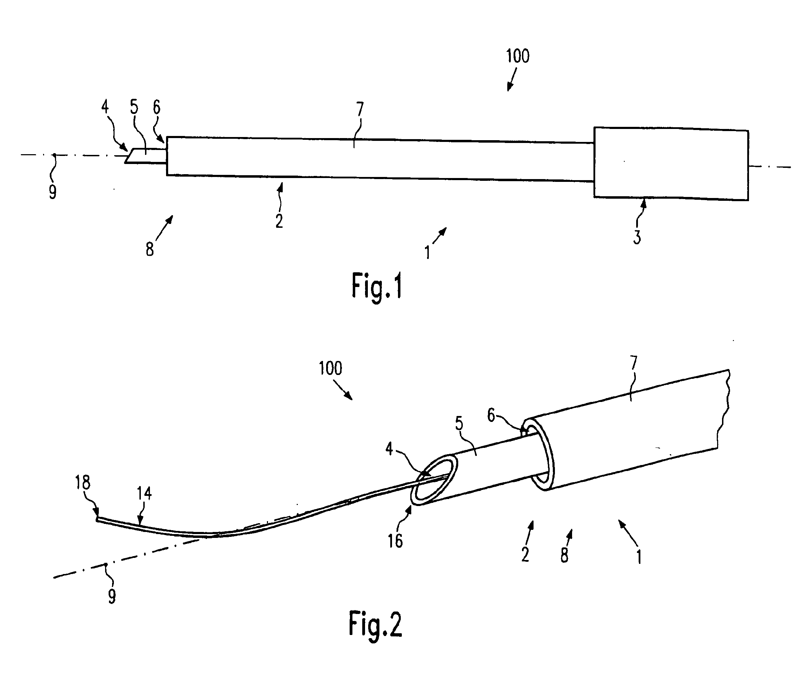

Figur 1 zeigt schematisch ein erfindungsgemäßes Frequenz-Ablationsgerät, nachfolgend kurz Ablationsgerät 1 genannt. Mit dem Ablationsgerät kann krankes Gewebe koaguliert werden. Das Ablationsgerät dieser Ausführungsform der Erfindung wird dazu im Hochfrequenzbereich betrieben.

Das Ablationsgerät 1 weist eine Rohreinheit 2 und eine damit verbundene Bedieneinheit 3 auf. Die Rohreinheit 2 hat koaxial ineinander gelagerte und mit Hilfe der Bedieneinheit 3 axial relativ zueinander bewegbare Rohre, von denen ein erstes Rohr 4 in einem zweiten Rohr 5 geführt ist, welches in einem dritten Rohr 6 geführt ist, das seinerseits in einem vierten Rohr 7 geführt ist. Das erste und dritte Rohr 4, 6 bestehen aus einem elektrisch leitenden federelastischen Material, z.B. Federstahl oder einer Ne-Ti-Legierung.

Durch das flexible, aus elektrisch isolierendem Material, z.B. einem Keramikoxidwerkstoff, bestehende zweite Rohr 5 sind das erste und das dritte Rohr 4, 6 voneinander isoliert. Auch das vierte Rohr 7 besteht aus einem flexiblen, elektrisch isolierenden Material, z.B. einem Keramikoxidwerkstoff. Durch die Flexibilität der Rohre 4, 5, 6, 7 ist auch die Rohreinheit 2 insgesamt flexibel.

In Figur 2 ist ein distaler Abschnitt 8 des in Figur 1 in der Ausgangsstellung 100 gezeigten Ablationsgerätes 1 vergrößert dargestellt. In der Ausgangsstellung 100 ist das erste Rohr 4 in sein führendes Rohr, also das zweite Rohr 5, zurückgezogen und das dritte Rohr 6 ist in sein führendes Rohr, also das vierte Rohr 7, zurückgezogen. Dabei ragt das zweite Rohr 4 in Richtung einer Längsachse 9 der Rohreinheit 2 ein Stück aus dem vierten Rohr 7 hervor.

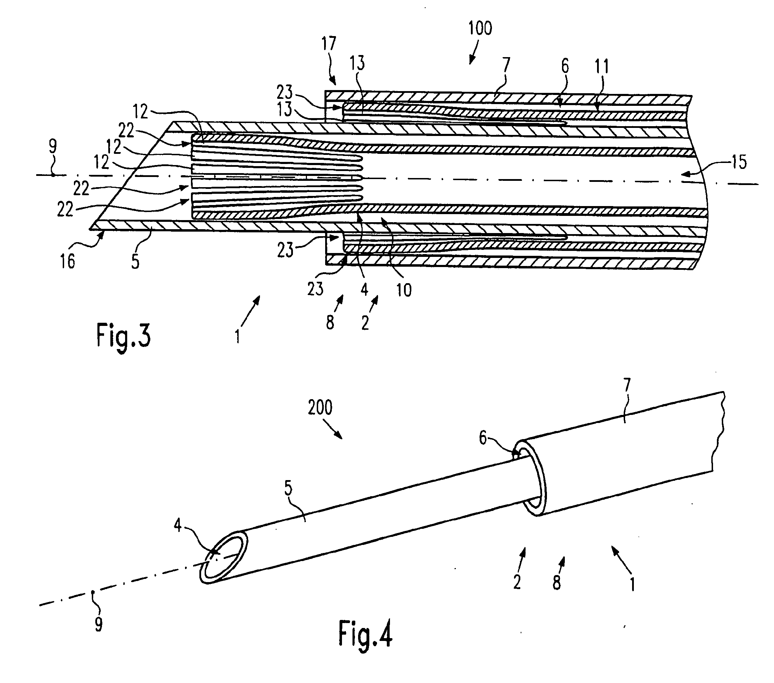

Figur 3 zeigt eine Schnittansicht des sich in Ausgangsstellung 100 befindenden distalen Endes 8 aus Figur 2 in einer nochmals vergrößerten und in Richtung der Längsachse 9 gestauchten Darstellung. Wie aus Figur 3 ersichtlich ist, weisen das erste Rohr 4 und das dritte Rohr 5 an ihren distalen Enden 10, 11 jeweils mehrere federelastisch verformbare, streifenartige Arme 12, 13 auf. Die Anzahl der Arme 12, 13 liegt vorzugsweise jeweils im Bereich von 4 bis 12.

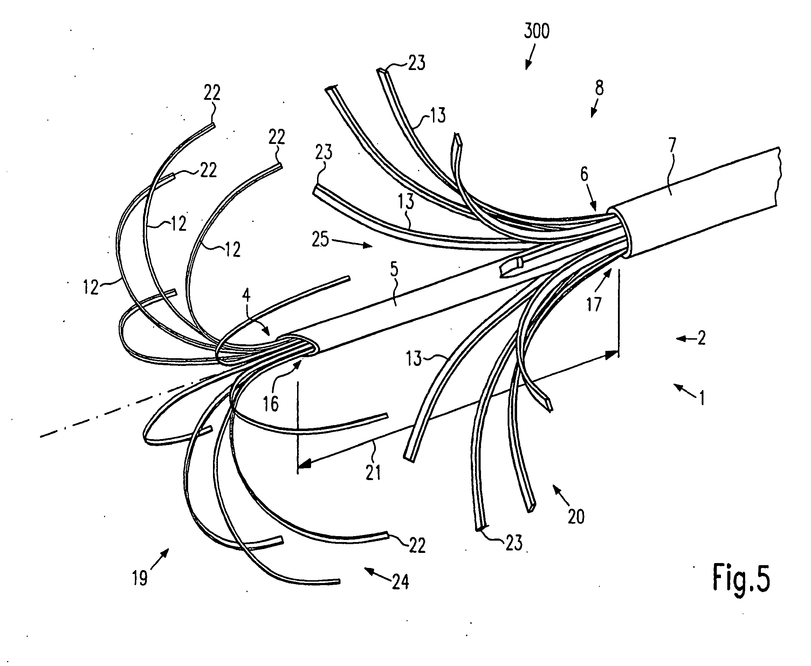

Die Arme 12, 13 sind jeweils einstückig mit ihrem Rohr 4, 6 durch Laserschneiden gespleißt ausgebildet und haben distale Endabschnitte 22, 23. In entspanntem Zustand nehmen die Arme 12, 13 in eine von einer axialen Mitte ihres jeweiligen Rohres 4, 6 abgespreizte Lage ein, wodurch sie jeweils eine schirmartige Elektrodenanordnung ausbilden, was mit Bezug auf Figur 5 noch näher erläutert wird. In der in Figur 3 gezeigten, in ihr jeweiliges führendes Rohr 5, 7 zurückgezogenen Position liegen die Arme 12, 13 an ihrem führendem Rohr 5, 7 nach außen federelastisch vorgespannt an.

In der Ausgangsstellung 100 wird das Ablationsgerät in einen zu behandelnden menschlichen oder tierischen Körper eingeführt. Dies kann mit Hilfe eines zuvor in den Körper eingebrachten Führungsdrahtes 14 erfolgen, wie dies in Figur 2 angedeutet ist. Das Ablationsgerät 1 wird über den Führungsdraht 14 geschoben, wobei der Führungsdraht 14 durch einen Innenraum 15 des ersten Rohres 4 verläuft.

Auf diese Weise wird das Ablationsgerät 1 auf der durch den Führungsdraht 14 vorbestimmten Bahn durch den zu behandelnden Körper geleitet. Dabei erleichtert die angeschrägte und scharfe Ausgestaltung eines distalen Endes 16 des zweiten Rohres 5 das Vordringen des Ablationsgerätes 1.

In einer alternativen Ausführungsform der vorliegenden Erfindung kann auch ein distales Ende 17 des vierten Rohres 7 scharf und/oder angeschrägt ausgeführt sein, um das Vordringen des Ablationsgerätes 1 weiter zu erleichtern.

In einer Weiterbildung der Erfindung kann der Führungsdraht 14 hohl ausgebildet sein, wodurch fließfähige Medien durch den Führungsdraht 14 in den zu behandelnden Körper einleitbar sind, und zwar unmittelbar in den Bereich, bis zu dem sich der Führungsdraht 14 mit seinem distalen Ende 18 im zu behandelnden Körper erstreckt. Auf diese Weise kann beispielsweise ein flüssiges Narkosemittel bereits während des Einführens des Führungsdrahtes 14 und/oder nach seinem erfolgten Einführen in den zu behandelnden Körper eingebracht werden. So wird das Narkosemittel unmittelbar den von Verletzungen und der Behandlung betroffenen Regionen des Körpers zugeführt.

Ist das Ablationsgerät 1 soweit in den Körper eingeführt, dass sich das distale Ende 17 des vierten Rohres 7 im Bereich vor dem zu behandelnden Gewebe befindet, wird das Ablationsgerät 1 von seiner Ausgangsstellung 100 in eine Zwischenstellung 200 gebracht, wie sie in Figur 4 dargestellt ist. Dazu wird das zweite Rohr 5 distal in Richtung der Längsachse 9 soweit relativ zum vierten Rohr 7 ausgefahren, bis sich das distale Ende 16 des zweiten Rohres 5 hinter dem zu behandelnden kranken Gewebe befindet. Das kranke Gewebe wird dabei von dem zweiten Rohr 5 durchdrungen.

Während des Ausfahrens des zweiten Rohres 5, also während des Variierens der axialen Lage des zweiten Rohres 5 relativ zum vierten Rohr 7, bleibt in dieser Ausführungsform der Erfindung die Relativlage des ersten Rohres 4 zum zweiten Rohr 5 und die Relativlage des dritten Rohres 6 zum vierten Rohr 7 bestehen. Folglich befinden sich das erste und dritte Rohr 4, 6 in der Zwischenstellung 200 noch in ihren zurückgezogenen Positionen gegenüber ihrem jeweiligen führenden Rohr 5, 7.

Anschließend werden das erste und dritte Rohr 4, 6 aus ihrem jeweiligen führenden Rohr 5, 7 abschnittsweise herausgeschoben, wodurch sich die Arme 12, 13 des ersten und dritten Rohres 4, 6 gewebedurchdringend von der axialen Mitte ihres Rohres 4,6 abspreizen und hierdurch jeweils eine schirmartige Elektrodenanordnung 19, 20 ausbilden. Während dieses Vorganges behalten das zweite und vierte Rohr 5, 7 ihre relative Lage zueinander bei. Durch die schirmartige Gestalt der Elektrodenanordnungen 19, 20 ergibt sich ein annähemd flächiger elektrischer Kontakt mit dem umliegenden Gewebe.

Sind die Elektrodenanordnungen 19, 20 ausgebildet, befindet sich das Ablationsgerät in seiner in Figur 5 gezeigten Anwendungsstellung 300. Dabei ist ein Abstand 21 zwischen den beiden Elektrodenanordnungen 19, 20 durch den Austritt des ersten und dritten Rohres 4, 6 aus ihrem jeweiligen führenden Rohr 5, 7 bestimmt. Anders ausgedrückt definiert der zwischen dem distalen Ende 16 des zweiten Rohres 5 und dem distalen Ende 17 des vierten Rohres 7 zum Zeitpunkt des Ausfahrens der Arme 12, 13 eingestellte Abstand 21 den Abstand 21 der Elektrodenanordnungen 19, 20 zueinander.

In Figur 5 sind die Arme 12 und 13 in ihrem entspannten Zustand gezeigt. Dabei sind die Arme 12 des ersten Rohres 4 stärker gekrümmt als die Arme 13 des zweiten Rohres 6. Außerdem sind die Arme 12 des ersten Rohres 4 etwa um 180° gebogen, während die Arme 13 des zweiten Rohres 6 etwa um 90° gebogen sind. Diese konkret beschriebene Geometrie (180°, 90°) kann in verschiedenen Ausführungsformen der Erfindung varüert werden, auch in dazwischen, darüber oder darunter liegende Winkelbereiche.

Daher unterscheidet sich die schirmartige Form der Elektrodenanordnung 19 des ersten Rohres 4 von der schirmartigen Form der Elektrodenanordnung 20 des dritten Rohres 6. Durch diese Ausbildung der Schirme sind die Endabschnitte 22, 23 der Arme 12, 13 einander im Wesentlichen zugewandt. Dabei liegt eine konkave Seite 24 der Elektrodenanordnung 19 des ersten Rohres 4 einer konvexen Seite 25 der Elektrodenanordnung 20 des zweiten Rohres 6 gegenüber. Hiermit ist ein gutes elektrisches Feld für die Behandlung erzeugbar, das aber anders gestaltet ist, als bei einer von der Erfindung abweichenden Gestaltung, bei welcher die konkaven Seiten zweier Elektrodenanordnungen einander gegenüberliegen.

Je intensiver und homogener das zwischen den Elektrodenanordnungen 19, 20 ausgebildete elektrische Feld ist, desto intensiver und homogener ist das in Folge des elektrischen Feldes in dem zu behandelnden Gewebe erzeugte thermische Feld. Je intensiver und homogener das erzeugte thermische Feld ist, desto besser ist das zu behandelnde Gewebe koagulierbar.

In einer alternativen Ausführungsform der Erfindung kann wenigstens eine der Elektrodenanordnungen wenigstens zwei schirmartige Teilelektrodenanordnungen aufweisen, wobei jede Teilelektrodenanordnung Arme eigener Ausgestaltung hat. Auf diese Weise können die Endabschnitte der Arme der Elektrodenanordnungen 19, 20 im Raum besser verteilt angeordnet werden, wodurch ein noch homogeneres Feld zwischen den beiden Elektrodenanordnungen ausbildbar ist.

Eine Möglichkeit, zwei schirmartige Teilelektrodenanordnungen auszubilden, ist, die Endabschnitte der Arme einer Elektrodenanordnung in unterschiedlichem radialem Abstand von der Längsachse 9 ihres Rohres vorzusehen. Dies kann durch Variieren der Krümmungen und/oder der Längen der Arme einer Elektrodenanordnung erfolgen.

Die vorangegangene Beschreibung bezieht sich insbesondere auf einen bipolaren Betrieb des erfindungsgemäßen Ablationsgerätes 1. Beim bipolaren Betrieb fungieren die Elektrodenanordnungen 19, 20 als Pole entgegengesetzter Ladung. Das Ablationsgerät 1 ist jedoch auch im monopolaren Betrieb einsetzbar, wobei eine weitere Elektrodenanordnung an einer Extremität des zu behandelnden Körpers vorgesehen wird.

Weil die Arme in entspanntem Zustand bogenförmig ausgebildet sind, sind sie beim Herausschieben aus ihrem jeweiligen führenden Rohr 5, 7 bestrebt, diesen Entspannungszustand wieder einzunehmen und bewegen sich demgemäß auf gekrümmten Bahnen durch das umliegende Gewebe. Hierbei wird das Durchdringen des Gewebes im Wesentlichen mit der in Längsrichtung in die Arme 12, 13 wirkenden Kraft bewerkstelligt. Die Arme 12, 13 sind an ihren distalen Enden 22, 23 spitz und/oder scharf, um das Durchdringen des Gewebes zu erleichtern.

Das Verbringen des Ablationsgerätes 1 von seiner Ausgangsstellung 100 in seine Zwischen- und Anwendungsstellung 200, 300 wird in dieser Ausführungsform der Erfindung mit Hilfe der Bedieneinheit 3 bewerkstelligt. Dabei weist die Bedieneinheit 3 einen Mechanismus auf, der ein Bewegen in die Anwendungsstellung 300, also das Ausbilden der schirmartigen Elektrodenanordnungen 19, 20, erst dann zulässt, wenn die Zwischenstellung 200 erreicht worden ist.

Der Innenraum 15 des ersten Rohres 4 kann genutzt werden, um fließfähige Medien und/oder Apparaturen unmittelbar dem Bereich zuzuführen, in dem sich die Öffnung des ersten Rohres 4 befindet, oder in dem sich die Öffnung des zweiten Rohres 5 befindet, wenn das erste Rohr 4 in das zweite Rohr 5 zurückgezogen ist. Damit ist der genannte Bereich schnell und ohne weitere Belastungen oder Verletzungen für den zu behandelnden Körper erreichbar. Dies verbessert die Ein- und Zugriffsmöglichkeiten an der zu behandelnden Stelle und verkürzt die Zeit des Eingriffs insgesamt.

Abgesehen von dem bereits erläuterten Führungsdraht 14 kann die durch den Innenraum 15 des ersten Rohres 4 führbare Apparatur eine Leitungsanordnung sein, durch welche ein fließfähiges Medium leitbar ist. Soweit wie die Leitungsanordnung durch den Innenraum 15 geführt ist, wird das erste Rohr von einem Kontakt mit dem fließfähigen Medium freigehalten. Soll mit der Leitungsanordnung ein von dem Ablationsgerät 1 beabstandeter Bereich in dem zu behandelnden Körper erreicht werden, muss der Körper nur im Bereich nach dem Ablationsgerät 1 von der Leitungsanordnung durchdrungen werden.

Die Leitungsanordnung kann mehrere einzelne Leitungen aufweisen. Hierdurch sind mehrere Medien unabhängig voneinander einbringbar und es werden Spülprozesse möglich.

Durch die Leitungsanordnung oder den Innenraum 15 selbst sind somit fließfähige Medien wie Betäubungsmittel, Medikamente, Salzlösung oder Alkohol präzise und schnell an die betreffenden Stellen im Körper bringbar. Auch kann auf diese Weise ein Gewebeklebstoff beim Herausnehmen der Leitungsanordnung und/oder des Ablationsgerätes in dem zerteilten Gewebe zurückgelassen werden, um dieses zu verschließen.

## Patentansprüche

1. Frequenz-Ablationsgerät (1), mit welchem krankes Gewebe koagulierbar ist, mit koaxial ineinander gelagerten und axial relativ zueinander bewegbaren Rohren, von denen ein erstes Rohr (4), welches in einem zweites Rohr (5) geführt ist, und ein drittes Rohr (6), welches in einem vierten Rohr (7) geführt ist, an ihren distalen Enden (10, 11) jeweils mehrere federelastisch verformbare Arme (12, 13) aufweisen, welche sich in entspanntem Zustand in einer von der axialen Mitte ihres jeweiligen Rohres (4, 6) abgespreizten Lage befinden und hierdurch jeweils eine schirmartige Elektrodenanordnung (19, 20) ausbilden, deren Abstand (21) zueinander durch den Austritt des ersten und dritten Rohres (4, 6) aus ihrem jeweiligen führenden Rohr (5, 7) bestimmt ist, wobei der Abstand der Elektrodenanordnungen (19, 20) durch Variieren der axialen Lage des zweiten Rohres (5) relativ zum vierten Rohr (7) einstellbar ist, wobei die Arme (12, 13) des ersten und/oder dritten Rohres (4, 6) einstückig mit ihrem Rohr (4, 6) und durch Laserschneiden gespleißt ausgebildet sind.

2. Frequenz-Ablationsgerät nach Anspruch 1,
**dadurch gekennzeichnet,**
**dass** eine Elektrodenanordnung wenigstens zwei schirmartige Teilelektrodenanordnungen aufweist, wobei jede Teilelektrodenanordnung Arme eigener Ausgestaltung aufweist.

3. Frequenz-Ablationsgerät nach Anspruch 2,
**dadurch gekennzeichnet,**
**dass** die Endabschnitte der Arme der ersten Teilelektrodenanordnung einen anderen radialen Abstand von einer Längsachse ihres Rohres haben als die Arme der zweiten Teilelektrodenanordnung.

4. Frequenz-Ablationsgerät nach wenigstens einem der vorhergehenden Ansprüche,
**dadurch gekennzeichnet,**
**dass** die Elektrodenanordnungen (19, 20) unterschiedlich bogenförmig gekrümmte Arme (12, 13) aufweisen und/oder wenigstens eine der Elektrodenanordnungen unterschiedlich bogenförmig gekrümmte Arme aufweist.

5. Frequenz-Ablationsgerät nach wenigstens einem der vorhergehenden Ansprüche,
**dadurch gekennzeichnet,**
**dass** das Ablationsgerät (1) eine Bedieneinheit (3) aufweist, mit welcher es von einer Ausgangsstellung (100), in welcher das erste und dritte Rohr (4, 6) in ihr jeweiliges führendes Rohr (5, 7) zurückgezogen sind, über eine Zwischenstellung (200), in der die axiale Lage des zweiten Rohres (5) relativ zum vierten Rohr (7) eingestellt ist und sich das erste und dritte Rohr (4, 6) noch in den genannten zurückgezogenen Positionen befinden, in eine Anwendungsstellung (300) bringbar ist, in welcher die Elektrodenanordnungen (19, 20) ausgebildet sind.

6. Frequenz-Ablationsgerät nach Anspruch 5,
**dadurch gekennzeichnet,**
**dass** die Elektrodenanordnungen (19, 20) erst dann schirmartig ausbildbar sind, wenn die Zwischenstellung (200) erreicht worden ist.

7. Frequenz-Ablationsgerät nach wenigstens einem der vorhergehenden Ansprüche,
**dadurch gekennzeichnet,**
**dass** durch den Innenraum (15) des ersten Rohres (4) ein fließfähiges Medium leitbar und/oder eine Apparatur (14) führbar ist.

8. Frequenz-Ablationsgerät nach Anspruch 7,
**dadurch gekennzeichnet,**
**dass** die Apparatur ein Führungsdraht (14) ist, an dem entlang das Ablationsgerät geführt in seine Verwendungsposition bringbar ist.

9. Frequenz-Ablationsgerät nach Anspruch 7,
**dadurch gekennzeichnet,**
**dass** die Apparatur eine Leitungsanordnung ist, durch welche ein fließfähiges Medium leitbar ist.

10. Frequenz-Ablationsgerät nach Anspruch 7 oder 9,
**dadurch gekennzeichnet,**
**dass** das fließfähige Medium ein Betäubungsmittel, ein Gewebeklebstoff oder ein Medikament ist.

## Claims

1. Frequency ablation device (1), with which diseased tissue may be coagulated, with tubes inserted coaxially within each other and axially displaceable relative to each other, of which a first tube (4), which is guided in a second tube (5), and a third tube (6),which is guided in a fourth tube (7), each have a plurality of elastically deformable arms (12, 13) at their distal ends (10, 11), which in relaxed condition are located in a position braced by the axial middle of their respective tube (4, 6) and hereby each form a screen-like electrode arrangement (19, 20), with the distance (21) between them being determined by the extension of the first and third tubes (4, 6) from the respective guiding tube (5, 7), wherein the distance between the electrode arrangements (19, 20) can be set by varying the axial position of the second tube (5) relative to the fourth tube (7), wherein the arms (12, 13) of the first and/or third tube (4, 6) are embodied in one piece with their tube (4, 6) and spliced by laser cutting.

2. Frequency ablation device according to claim 1,
**characterised in**
**that** an electrode arrangement comprises at least two screen-like partial electrode arrangements, wherein each partial electrode arrangement comprises arms with their own embodiment.

3. Frequency ablation device according to claim 2,
**characterised in**
**that** the end portions of the arms of the first partial electrode arrangement have a different radial distance from a longitudinal axis of their tube than the arms of the second partial electrode arrangement.

4. Frequency ablation device according to at least one of the preceding claims,
**characterised in**
**that** the electrode arrangements (19, 20) comprises differently curved arcuate arms (12, 13) and/or at least one of the electrode arrangements comprises differently curved arcuate arms.

5. Frequency ablation device according to at least one of the preceding claims,
**characterised in**
**that** the ablation device (1) comprises a control unit (3), with which it may be brought from an initial position (100), in which the first and third tube (4, 6) are retracted into their respective guiding tubes (5, 7), via an intermediate position (200), in which the axial position of the second tube (5) is set relatively to the fourth tube (7) and the first and third tubes (4, 6) are still in said retracted positions, into an application position (300), in which the electrode arrangements (19, 20) are embodied.

6. Frequency ablation device according to claim 5,
**characterised in**
**that** the electrode arrangements (19, 20) can only be embodied as screens when the intermediate position (200) is reached.

7. Frequency ablation device according to at least one of the preceding claims,
**characterised in**
**that** a flowable medium can be fed and/or an apparatus (14) can be guided through the interior (15) of the first tube (4).

8. Frequency ablation device according to claim 7,
**characterised in**
**that** the apparatus is a guide wire (14) along which the ablation device is guided to bring it into its position of use.

9. Frequency ablation device according to claim 7,
**characterised in**
**that** the apparatus is a line arrangement through which a flowable medium may be fed.

10. Frequency ablation device according to claim 7 or 9,
**characterised in**
**that** the flowable medium is an anaesthetic, tissue adhesive or a drug.

## Revendications

1. Appareil d'ablation à fréquence (1) avec lequel un tissu malade est apte à coaguler, avec des tubes placés coaxialement les uns dans les autres et mobiles axialement les uns par rapport aux autres, un premier tube (4) qui est guidé dans un deuxième tube (5), et un troisième tube (6) qui est guidé dans un quatrième tube (7) présentant à leurs extrémités distales (10, 11) plusieurs bras déformables élastiquement (12, 13) qui, en position détendue, se trouvent dans une position écartée par rapport au centre axial de leurs tubes (4, 6) et forment ainsi des dispositifs à électrodes en forme de parapluies (19, 20) dont l'écartement mutuel (21) est défini par la sortie des premier et troisième tubes (4, 6) hors de leurs tubes de guidage respectifs (5, 7), l'écartement des dispositifs à électrodes (19, 20) étant réglable grâce à une variation de la position axiale du deuxième tube (5) par rapport au quatrième tube (7), et les bras (12, 13) du premier et/ou troisième tube (4, 6) étant réalisés d'une seule pièce avec leur tube (4, 6) et épissurés par coupe au laser.

2. Appareil d'ablation à fréquence selon la revendication 1, **caractérisé en ce qu'**un dispositif à électrodes comprend au moins deux dispositifs à électrodes partiels en forme de parapluies, chacun de ces dispositifs partiels présentant des bras d'une configuration propre.

3. Appareil d'ablation à fréquence selon la revendication 2, **caractérisé en ce que** les parties d'extrémité des bras du premier dispositif à électrodes partiel ont un écartement radial, par rapport à un axe longitudinal de leur tube, qui est différent de celui des bras du second dispositif à électrodes partiel.

4. Appareil d'ablation à fréquence selon l'une au moins des revendications précédentes, **caractérisé en ce que** les dispositifs à électrodes (19, 20) présentent des bras (12, 13) avec des courbures différentes et/ou l'un au moins des dispositifs à électrodes présente des bras avec des courbures différentes.

5. Appareil d'ablation à fréquence selon l'une au moins des revendications précédentes, **caractérisé en ce que** l'appareil d'ablation (1) comporte une unité de manipulation (3) avec laquelle il est apte à être amené d'une position de départ (100) dans laquelle les premier et troisième tubes (4, 6) sont rétractés dans leurs tubes de guidage respectifs (5, 7), à une position d'utilisation (300) dans laquelle les dispositifs à électrodes (19, 20) sont formés, en passant par une position intermédiaire (200) dans laquelle la position axiale du deuxième tube (5) par rapport au quatrième tube (7) est réglée et les premier et troisième tubes (4, 6) se trouvent encore dans les positions rétractées mentionnées.

6. Appareil d'ablation à fréquence selon la revendication 5, **caractérisé en ce que** les dispositifs à électrodes (19, 20) ne sont aptes à prendre une forme de parapluie qu'une fois que la position intermédiaire (200) a été atteinte.

7. Appareil d'ablation à fréquence selon l'une au moins des revendications précédentes, **caractérisé en ce qu'**un produit fluide est apte à passer et/ou un appareillage (14) est apte à être guidé dans l'intérieur (15) du premier tube (4).

8. Appareil d'ablation à fréquence selon la revendication 7, **caractérisé en ce que** l'appareillage est constitué par un fil de guidage (14) le long duquel l'appareil d'ablation est apte à être guidé jusqu'à sa position d'utilisation.

9. Appareil d'ablation à fréquence selon la revendication 7, **caractérisé en ce que** l'appareillage est constitué par un dispositif de conduit par lequel un produit fluide peut passer.

10. Appareil d'ablation à fréquence selon la revendication 7 ou 9, **caractérisé en ce que** le produit liquide est constitué par un anesthésiant, une colle pour tissus ou un médicament.
